# EUROPEAN PATENT APPLICATION

(11) **EP 4 596 072 A1**
(43) Date of publication of application: **06.08.2025**
(21) Application number: 23872388.6
(22) Date of filing: 27.09.2023
(51) Int. Cl.: A63H 11/00, A63H 3/00

(54) **ROBOT**

(30) Priority: 29.09.2022 JP 2022156760
(71) Applicant: Nitto Denko Corporation, Ibaraki-shi, Osaka 567-8680 (JP)
(72) Inventor: FUKUSHIMA, Rihito, Ibaraki-shi, Osaka 567-8680 (JP); YAMAUCHI, Kengo, Ibaraki-shi, Osaka 567-8680 (JP); KIYOSHIMA, Keita, Ibaraki-shi, Osaka 567-8680 (JP); SHIMIZU, Yusuke, Ibaraki-shi, Osaka 567-8680 (JP)
(74) Representative: Epping - Hermann - Fischer
(86) International application number: PCT/JP2023/035069
(87) International publication number: WO 2024/071166

(57) **Abstract**

A robot configured to naturally guide breathing for a user is provided. The robot according to one aspect of the present invention is a robot configured to guide breathing for a user, and includes: an exterior member; a first detector configured to acquire information on the breathing of the user; and a controller configured to control an operation of the robot so as to guide the breathing of the user to a predetermined state based on the information acquired by the first detector.

## Description

### TECHNICAL FIELD

The present disclosure relates to a robot.

### BACKGROUND

Conventionally, a robot configured to provide comfort to a user by interacting with a user is known (for example, see Patent Document 1).

With respect to the above, a breathing guidance device configured to guide breathing for a user to a predetermined state in order to relax the user to activate a parasympathetic nervous system of the user is known. For example, Patent Document 2 discloses a device including a respiratory sensor that is disposed in a body formed in a huggable shape and that is configured to detect the respiratory rate of a user holding the body, an expansion-contraction mechanism that is built in the body and that is configured to expand and contract the contact side of the body with the user holding the body, and an expansion-contraction control unit configured to drive and control the expansion-contraction mechanism.

### Related Art Document

### Patent Document

[Patent Document 1] International Publication Pamphlet No. WO 2017/169826
[Patent Document 2] Japanese Laid-open Patent Publication No. 2013-022302

### SUMMARY OF THE INVENTION

### Problem to be solved by the invention

In a robot configured to provide comfort to a user, it is required to naturally guide the breathing for a user in order to relax the user.

It is an object of the present invention to provide a robot configured to naturally guide breathing for a user.

### Means for Solving the Problem

A robot according to an embodiment of the present invention is a robot configured to guide breathing for a user, and includes an exterior member; a first detector configured to acquire information on the breathing of the user; and a controller configured to control an operation of the robot so as to guide the breathing of the user to a predetermined state based on the information acquired by the first detector.

### Effect of the invention

According to the present invention, a robot that can naturally guide breathing for a user can be provided.

### BRIEF DESCRIPTION OF THE DRAWINGS

[FIG. 1] FIG. 1 is a perspective view illustrating a robot according to an embodiment.
[FIG. 2] FIG. 2 is a side view of the robot of FIG. 1.
[FIG. 3] FIG. 3 is a cross-sectional view along the III-III cut line in FIG. 2.
[FIG. 4] FIG. 4 is a view illustrating a configuration of a camera according to the embodiment.
[FIG. 5] FIG. 5 is a view illustrating a configuration of a vital sensor according to the embodiment.
[FIG. 6] FIG. 6 is a view illustrating a configuration of an expansion-contraction mechanism according to the embodiment.
[FIG. 7] FIG. 7 is a block diagram illustrating a hardware configuration of a controller according to the embodiment.
[FIG. 8] FIG. 8 is a block diagram illustrating a functional configuration of the controller according to a first embodiment.
[FIG. 9] FIG. 9 is a view illustrating a state of breathing guidance by a robot according to the first embodiment.
[FIG. 10] FIG. 10 is a flowchart illustrating a process by the controller according to the first embodiment.
[FIG. 11] FIG. 11 is a block diagram illustrating a functional configuration of a controller according to a second embodiment.
[FIG. 12] FIG. 12 is a flowchart illustrating a process by the controller according to the second embodiment.
[FIG. 13] FIG. 13 is a view illustrating an example arrangement of an expansion-contraction mechanism in a robot according to a third embodiment.
[FIG. 14] FIG. 14 is a view illustrating an example configuration of the expansion-contraction mechanism in the robot according to the third embodiment.
[FIG. 15] FIG. 15 is a block diagram illustrating a functional configuration of the controller according to the third embodiment.
[FIG. 16] FIG. 16 is a flowchart illustrating a process by the controller according to the third embodiment.

### DESCRIPTION OF THE EMBODIMENTS

In the following, embodiments of the present disclosure will be described in detail with reference to the drawings. In the drawings, the same components are denoted by the same reference symbols, and duplicated description will be appropriately omitted.

The embodiments described below are examples of a robot for embodying the technical idea of the present disclosure, and the present disclosure is not limited to the embodiments described below. The dimensions, materials, shapes, relative arrangements, and the like of the components described below are intended to illustrate the examples, not to limit the scope of the present disclosure thereto, unless otherwise specified. Additionally, the size, positional relationship, and the like of members illustrated in the drawings may be exaggerated for clarity of description.

### <Overall Configuration Example of Robot 100>

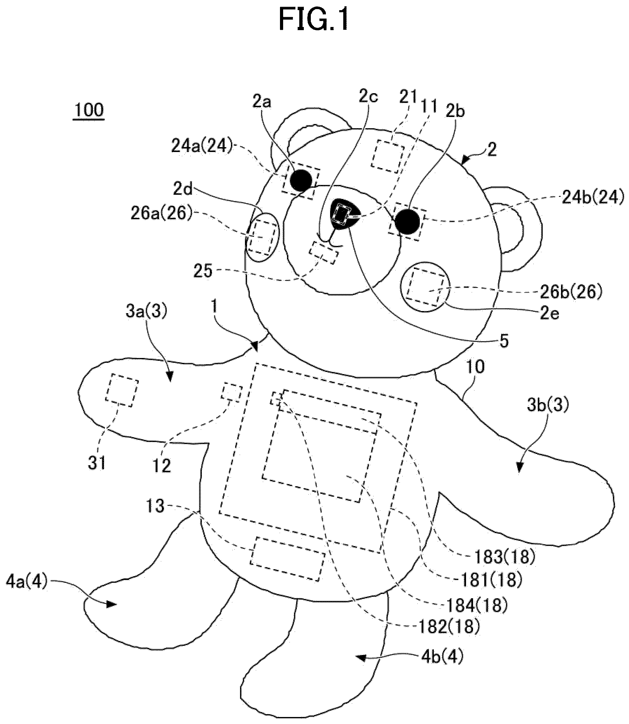

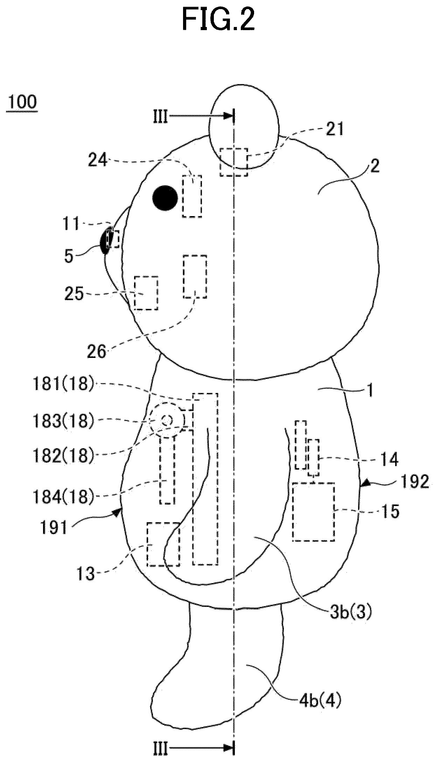

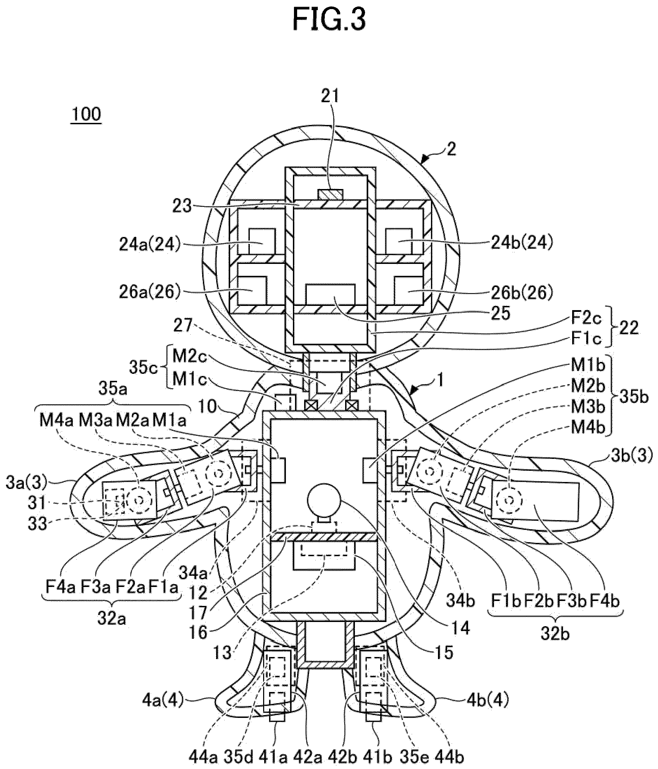

A configuration of a robot 100 according to an embodiment will be described with reference to FIGS. 1 to 3. FIG. 1 is a perspective view illustrating the robot 100 according to the embodiment. FIG. 2 is a side view of the robot 100. FIG. 3 is a cross-sectional view along a cutting line III-III in FIG. 2.

The robot 100 is a robot that includes exterior members 10 and that is configured to be driven by supplied power. The robot 100 described in the present specification as an example is a doll-type communication robot in the shape of a bear cub. The robot 100 is manufactured to have a size and weight suitable for being held by a user. Here, the user indicates a user of the robot 100. Typical examples of the user include a working adult living alone, a senior person whose child has moved out, and a frail elderly person who is a target of home healthcare. Here, the user may include a contact person who simply contacts the robot 100, such as an administrator of the robot 100, in addition to the user of the robot 100. Additionally, in the present specification, the term "hold" may be referred to as "carry".

In the present embodiment, the robot 100 can guide breathing for the user. The user corresponds to a "person" who breathes. Breathing is the only system that can be consciously changed from the outside in the autonomic nervous system of the "person". For example, the robot 100 can cause the user to feel a slow respiratory rhythm to guide the user's respiratory rhythm to this rhythm and synchronize to the rhythm. Respiratory properties that can be guided by the robot 100 include the respiratory rate, the respiratory frequency, the respiratory count, the respiratory depth, the respiratory amplitude, and the like. The robot 100 can activate the parasympathetic nervous system of the user and relax the user by guiding the breathing of the user to a predetermined respiratory property.

The exterior member 10 may have flexibility. The exterior member 10 contains, for example, a soft material that is comfortable to touch when the user of the robot 100 touches the robot 100. The exterior member 10 may have flexibility by containing at least one of an elastic material or a porous material. Specifically, as the material of the exterior member 10, a material containing an organic material, such as urethane foam, rubber, resin, or fiber can be used. The exterior member 10 preferably includes an exterior, such as a urethane foam material, having a heat insulating property, and a soft cloth material covering the outer surface of the exterior. The exterior member 10 has flexibility, for example, by containing at least one of an elastic material or a porous material, so that the user can feel the softness of the robot 100. With this, a restraining feeling and user resistance can be reduced, thereby promoting communication between the user and the robot 100.

The robot 100 includes, for example, a trunk 1, a head 2, arms 3, and legs 4. The head 2 has a right eye 2a, a left eye 2b, a mouth 2c, a right cheek 2d, and a left cheek 2e. The arms 3 include a right arm 3a and a left arm 3b, and the legs 4 include a right leg 4a and a left leg 4b. Here, the trunk 1 corresponds to a robot main body. As illustrated in FIG. 2, a portion of the trunk 1 of the robot 100 on the side where a nose 5 of the robot 100 is disposed is an abdomen 191 of the robot 100. Additionally, a portion of the trunk 1 of the robot 100 on the side opposite to the side where the nose 5 of the robot 100 is disposed is a back 192 of the robot 100.

As illustrated in FIGS. 1 to 3, the head 2, the arm 3, and the leg 4 correspond to a drive section connected to the robot main body so as to be relatively displaceable. The drive section in the present embodiment includes the arm 3 connected to the robot main body in the robot 100 so as to be relatively displaceable. The robot 100 can guide breathing for the user by controlling at least one of an operation of wrapping the arm 3 around a part of the user's body, an operation of pressurizing the user's skin by the arm 3, or an operation of stroking a part of the user's body by the arm 3.

In the present embodiment, the arm 3 is configured to be displaceable with respect to the trunk 1. For example, when the robot 100 is held by the user, the robot 100 displaces the right arm 3a and the left arm 3b to contact a neck, a trunk, or the like of the user so as to hold the user. This operation allows the user to feel an affinity for the robot 100, and thus promotes the interaction between the user and the robot 100. Here, the interaction with the user indicates an action of the user and the robot 100 touching each other (a contact action), such as rubbing, tapping (touching), hugging (embracing), and the like.

The trunk 1, the head 2, the arms 3, and the legs 4 are all covered with the exterior members 10. The exterior member at the trunk 1 and the exterior member at the arm 3 are integrated, and the exterior members at the head 2 and the leg 4 are separated from the exterior member at the trunk 1 and the arm 3. However, the embodiments are not limited to these configurations, and for example, only a portion of the robot 100 that is likely to be contacted by the user may be covered by the exterior member 10. Additionally, at least one of the exterior members 10 at the trunk 1, the head 2, the arm 3, and the leg 4 may be separated from the other exterior members. Further, a portion in the head 2, the arm 3, and the leg 4 that does not displace may be configured only by the exterior member 10 without including a component such as a sensor therein.

The robot 100 includes a camera 11, a tactile sensor 12, a controller 13, a vital sensor 14, a battery 15, an expansion-contraction mechanism 18, a first capacitive sensor 21, and a second capacitive sensor 31 inside the exterior members 10. Additionally, the robot 100 includes the tactile sensor 12, the controller 13, the vital sensor 14, the battery 15, and the expansion-contraction mechanism 18 inside the exterior member 10 at the trunk 1. Additionally, the robot 100 includes the camera 11, the first capacitive sensor 21 inside the exterior member 10 at the head 2, and the second capacitive sensor 31 inside the exterior member 10 at the arm 3.

Additionally, the robot 100 includes displays 24, a speaker 25, and lights 26 inside the exterior member 10 at the head 2. Additionally, the robot 100 includes the displays 24 inside the exterior member 10 at the right eye 2a and the left eye 2b. Additionally, the robot 100 includes the speaker 25 inside the exterior member 10 at the mouth 2c, and the lights 26 inside the exterior member 10 at the right cheek 2d and the left cheek 2e.

More specifically, as illustrated in FIG. 3, the robot 100 includes a trunk frame 16 and a trunk mounting base 17 inside the exterior member 10 at the trunk 1. Additionally, the robot 100 includes a head frame 22 and a head mounting base 23 inside the exterior member 10 at the head 2. Further, the robot 100 includes a right arm frame 32a and a right arm mounting base 33 inside the exterior member 10 at the right arm 3a, and a left arm frame 32b inside the exterior member 10 at the left arm 3b. In addition, the robot 100 includes a right leg frame 42a inside the exterior member 10 at the right leg 4a, and a left leg frame 42b inside the exterior member 10 at the left leg 4b.

The trunk frame 16, the head frame 22, the right arm frame 32a, the left arm frame 32b, the right leg frame 42a, and the left leg frame 42b are structures each formed by combining multiple columnar members. The trunk mounting base 17, the head mounting base 23, and the right arm mounting base 33 are plate members having placement surfaces. The trunk mounting base 17 is fixed to the trunk frame 16, the head mounting base 23 is fixed to the head frame 22, and the right arm mounting base 33 is fixed to the right arm frame 32a. Here, the trunk frame 16, the head frame 22, the right arm frame 32a, the left arm frame 32b, the right leg frame 42a, and the left leg frame 42b may be formed in a box shape including multiple plate members.

The right arm frame 32a is connected to the trunk frame 16 via a right arm connection mechanism 34a, and is driven by a right arm servo motor 35a, thereby being relatively displaceable with respect to the trunk frame 16. The displacement of the right arm frame 32a causes the right arm 3a to displace relative to the trunk 1. The right arm connection mechanism 34a preferably includes a reduction gear that increases the output torque of the right arm servo motor 35a, for example.

In the present embodiment, the right arm frame 32a is configured by a multi-joint robot arm including multiple frame members and multiple connection mechanisms. For example, the right arm frame 32a includes a right shoulder frame F1a, a right upper arm frame F2a, a right elbow frame F3a, and a right forearm frame F4a. The trunk frame 16, the right shoulder frame F1a, the right upper arm frame F2a, the right elbow frame F3a, and the right forearm frame F4a are connected to each other via respective connection mechanisms.

The right arm servo motor 35a is a generic term of multiple servo motors. For example, the right arm servo motor 35a includes a right shoulder servo motor M1a, a right upper arm servo motor M2a, a right elbow servo motor M3a, and a right forearm servo motor M4a. The right shoulder servo motor M1a rotates the right shoulder frame F1a about a rotation axis perpendicular to the trunk frame 16. The right upper arm servo motor M2a rotates the right upper arm frame F2a about a rotation axis perpendicular to a rotation axis of the right shoulder frame F1a. The right elbow servo motor M3a rotates the right elbow frame F3a about a rotation axis perpendicular to a rotation axis of the right upper arm frame F2a. The right forearm servo motor M4a rotates the right forearm frame F4a about a rotation axis perpendicular to a rotation axis of the right elbow frame F3a.

The left arm frame 32b is connected to the trunk frame 16 via a left arm connection mechanism 34b, and is driven by a left arm servo motor 35b, thereby being relatively displaceable with respect to the trunk frame 16. The displacement of the left arm frame 32b causes the left arm 3b to displace relative to the trunk 1. The left arm connection mechanism 34b preferably includes a reduction gear that increases the output torque of the left arm servo motor 35b, for example.

In the present embodiment, the left arm frame 32b is configured by a multi-joint robot arm including multiple frame members and multiple connection mechanisms. For example, the left arm frame 32b includes a left shoulder frame F1b, a left upper arm frame F2b, a left elbow frame F3b, and a left forearm frame F4b. The trunk frame 16, the left shoulder frame F1b, the left upper arm frame F2b, the left elbow frame F3b, and the left forearm frame F4b are connected to each other via respective connection mechanisms.

The left arm servo motor 35b is a generic term of multiple servo motors. For example, the left arm servo motor 35b includes a left shoulder servo motor M1b, a left upper arm servo motor M2b, a left elbow servo motor M3b, and a left forearm servo motor M4b. The left shoulder servo motor M1b rotates the left shoulder frame F1b about a rotation axis perpendicular to the trunk frame 16. The left upper arm servo motor M2b rotates the left upper arm frame F2b about a rotation axis perpendicular to a rotation axis of the left shoulder frame F1b. The left elbow servo motor M3b rotates the left elbow frame F3b about a rotation axis perpendicular to a rotation axis of the left upper arm frame F2b. The left forearm servo motor M4b rotates the left forearm frame F4b about a rotation axis perpendicular to a rotation axis of the left elbow frame F3b. As described, the arm 3 includes the four-axis joints, thereby enabling the robot 100 to realize a realistic operation. The realistic operation refers to a natural motion as a motion of animals including humans. In the present embodiment, the realistic operation corresponds to a natural motion of the robot 100 as a bear cub.

The head frame 22 is connected to the trunk frame 16 via a head connection mechanism 27, and is driven by a head servo motor 35c, thereby being relatively displaceable with respect to the trunk frame 16. The displacement of the head frame 22 causes the head 2 to displace relative to the trunk 1. The head connection mechanism 27 preferably includes a reduction gear that increases the output torque of the head servo motor 35c, for example.

In the present embodiment, the head frame 22 includes a neck frame F1c and a face frame F2c. The trunk frame 16, the neck frame F1c, and the face frame F2c are connected to each other via respective connection mechanisms.

The head servo motor 35c is a generic term of multiple servo motors. For example, the head servo motor 35c includes a neck servo motor M1c and a face servo motor M2c. The neck servo motor M1c rotates the neck frame F1c about a rotation axis perpendicular to the trunk frame 16. The face servo motor M2c rotates the face frame F2c around a rotation axis perpendicular to a rotation axis of the neck frame F1c. As described, the robot 100 can realize a more realistic operation because the head 2 includes the two-axis joints.

The right leg frame 42a is connected to the trunk frame 16 via a right leg connection mechanism 44a, and includes a right leg wheel 41a on the bottom side. In order to stabilize the posture of the robot 100, the robot 100 preferably includes two right leg wheels 41a in the front-rear direction of the right leg frame 42a. The right leg wheel 41a is driven by a right leg servo motor 35d, thereby being rotatable about a rotation axis perpendicular to the front-rear direction of the right leg frame 42a. The rotation of the right leg wheel 41a enables the robot 100 to travel. The right leg connection mechanism 44a preferably includes a reduction gear that increases the output torque of the right leg servo motor 35d, for example.

The left leg frame 42b is connected to the trunk frame 16 via a left leg connection mechanism 44b, and includes a left leg wheel 41b on the bottom side. In order to stabilize the posture of the robot 100, the robot 100 preferably includes two left leg wheels 41b in the front-rear direction of the left leg frame 42b. The left leg wheel 41b is driven by the left leg servo motor 35e, thereby being rotatable about a rotation axis perpendicular to the front-rear direction of the left leg frame 42b. The rotation of the left leg wheel 41b enables the robot 100 to travel. The left leg connection mechanism 44b preferably includes a reduction gear that increases the output torque of a left leg servo motor 35e, for example.

In the present embodiment, the robot 100 moves forward or backward by simultaneously rotating the right leg wheel 41a and the left leg wheel 41b forward or backward. The robot 100 turns right or left by braking one of the right leg wheel 41a or the left leg wheel 41b with a brake and turning the other forward or backward. As described, the robot 100 can realize a more realistic operation by the leg 4.

The tactile sensor 12, the controller 13, the vital sensor 14, and the battery 15 are fixed to the trunk mounting base 17. The controller 13 and the battery 15 are fixed to the side of the trunk mounting base 17 opposite to the side to which the tactile sensor 12 and the vital sensor 14 are fixed. Here, the arrangement of the controller 13 and the battery 15 is due to the space available on the trunk mounting base 17 and is not limited to the above described arrangement. However, if the battery 15 is fixed to the side of the trunk mounting base 17 opposite to the side to which the tactile sensor 12 and the vital sensor 14 are fixed, the center of gravity of the robot 100 is lowered because the battery 15 is heavier than other components. The center of gravity of the robot 100 is preferably low because at least one of the position or the posture of the robot 100 is stabilized and at least one of charging or replacement of the battery 15 is easily performed.

The first capacitive sensor 21 is fixed to the head mounting base 23, and the second capacitive sensor 31 is fixed to the right arm mounting base 33. The display 24 includes a right-eye display 24a and a left-eye display 24b. The right-eye display 24a, the left-eye display 24b, and the speaker 25 are fixed to the head frame 22. The lights 26 include a right-cheek light 26a and a left-cheek light 26b. The right-cheek light 26a and the left-cheek light 26b are fixed to the head frame 22.

Here, the tactile sensor 12, the controller 13, the vital sensor 14, the battery 15, the first capacitive sensor 21, the second capacitive sensor 31, and the like can be fixed by a screw member, an adhesive member, or the like. Additionally, the right-eye display 24a, the left-eye display 24b, the speaker 25, the right-cheek light 26a, the left-cheek light 26b, and the like can also be fixed by screw members, adhesive members, or the like.

The materials of the trunk frame 16, the trunk mounting base 17, the head frame 22, the head mounting base 23, the right arm frame 32a, the right arm mounting base 33, and the left arm frame 32b are not particularly limited, and a resin material, a metallic material, or the like can be used. However, from the viewpoint of ensuring strength during driving, it is preferable to use a metallic material, such as aluminum, for the trunk frame 16, the right arm frame 32a, and the left arm frame 32b. If the strength can be obtained, it is preferable to use a resin material for the material of each of these parts in order to reduce the weight of the robot 100. The materials of the trunk mounting base 17, the head frame 22, the head mounting base 23, the right arm mounting base 33, and the left arm frame 32b are not particularly limited, and a resin material or a metallic material can be used. However, from the viewpoint of reducing the weight of the robot 100, it is preferable to use a resin material.

The controller 13 controls an entire operation of the robot 100. In the present embodiment, the controller 13 can control the operation of the robot 100 so as to guide the breathing of the user to a predetermined state based on an output from the camera 11. The controller 13 is communicably connected to each of the camera 11, the tactile sensor 12, the vital sensor 14, the first capacitive sensor 21, the second capacitive sensor 31, the right arm servo motor 35a, and the left arm servo motor 35b by wire or wirelessly. Additionally, the controller 13 is communicably connected to each of the head servo motor 35c, the right leg servo motor 35d, and the left leg servo motor 35e by wire or wirelessly. Further, the controller 13 is communicably connected to each of the right-eye display 24a, the left-eye display 24b, the speaker 25, the right-cheek light 26a, and the left-cheek light 26b by wire or wirelessly.

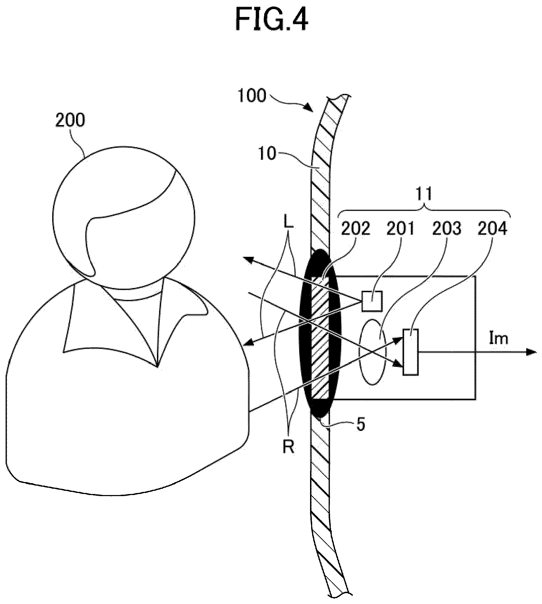

The camera 11 is an image sensor configured to output a captured image of the surroundings of the robot 100 to the controller 13. The camera 11 is an example of a capturing section configured to capture an image of the user. Additionally, the camera 11 is an example of a first detector configured to acquire information on the breathing of the user. Furthermore, the camera 11 is an example of an image sensor configured to acquire information on the breathing of the user based on the captured image of the user. The camera 11 is disposed at a position corresponding to the nose 5 of the bear cub inside the exterior member 10. The camera 11 can be fixed by an adhesive member or the like. Here, a configuration of the camera 11 will be described in detail with reference to FIG. 4, separately.

The tactile sensor 12 is a sensor element configured to acquire information felt by a tactile sense inherent in a human hand or the like, convert the information into a tactile signal, which is an electrical signal, and output the tactile signal to the controller 13. For example, the tactile sensor 12 converts information on pressure or vibration generated by the user contacting the robot 100 into a tactile signal by a piezoelectric element, and outputs the tactile signal to the controller 13. The tactile signal output from the tactile sensor 12 is used to detect whether a user has come into contact with or come in proximity to the robot 100.

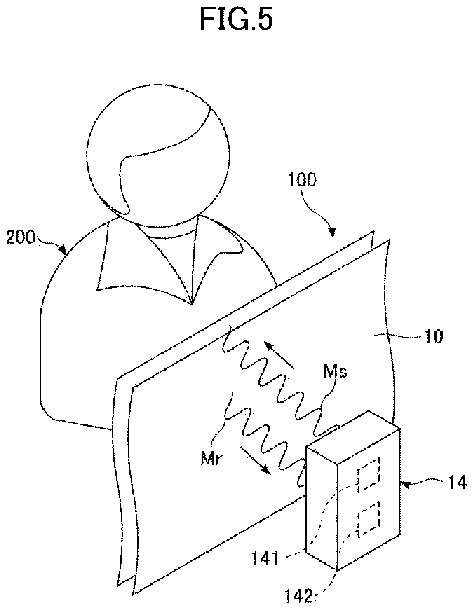

The vital sensor 14 is an example of the first detector configured to acquire the information on the breathing of the user. Additionally, the vital sensor 14 is an example of an electromagnetic wave sensor configured to acquire the information on the breathing of the user, using electromagnetic waves. Additionally, the vital sensor 14 is an example of a second detector configured to acquire information on at least one of a pulse, a heartbeat, a blood pressure, or a pulse pressure of the user. The vital sensor 14 may be disposed inside the exterior member 10 of the robot 100. Here, a configuration of the vital sensor 14 will be described in detail separately with reference to FIG. 5.

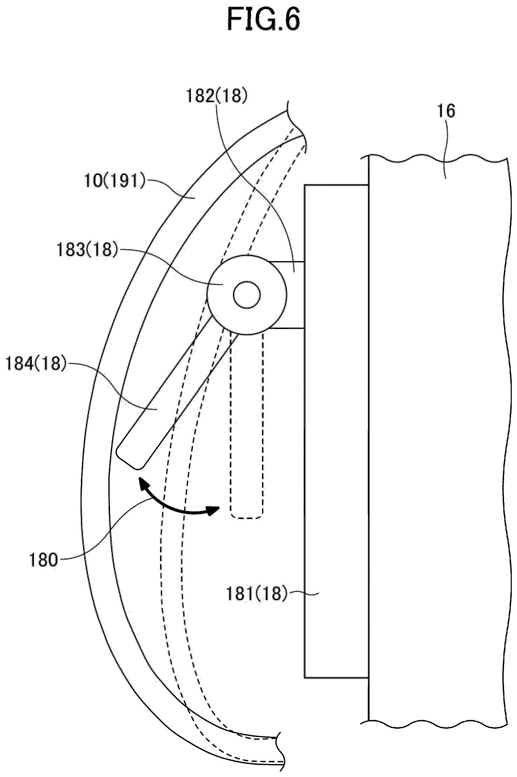

The expansion-contraction mechanism 18 is a mechanism that can expand and contract the trunk 1 of the robot 100. In the present embodiment, the expansion-contraction mechanism 18 is used to guide breathing for the user. Here, a configuration of the expansion-contraction mechanism 18 will be described in detail separately with reference to FIG. 6.

The first capacitive sensor 21 and the second capacitive sensor 31 are sensor elements configured to output, to the controller 13, a capacitance signal obtained by detecting, based on a change in capacitance, that the user has come into contact with or come in proximity to the robot 100. The first capacitive sensor 21 is preferably a rigid sensor having no flexibility in terms of stabilization of the exterior member 10. The arm 3 is a part that is easily touched by the user, and thus the second capacitive sensor 31 is preferably a sensor having flexibility including a conductive thread or the like from the viewpoint of improving the touch feeling. The capacitance signals output from the first capacitive sensor 21 and the second capacitive sensor 31 are used to detect that the user has contacted or approached the robot 100.

The right-eye display 24a and the left-eye display 24b are display modules configured to display character strings, such as characters, numerals, and symbols, or images in response to a command from the controller 13. The right-eye display 24a and the left-eye display 24b are configured by, for example, liquid crystal display modules. The character strings or images displayed on the right-eye display 24a and the left-eye display 24b may be used to express the emotion of the robot 100 or the like. For example, the robot 100 can implicitly induce the interaction with the user by displaying "smiling" images on the right-eye display 24a and the left-eye display 24b toward the user who is sitting feeling happy, to empathize with the user's happiness.

The speaker 25 is a speaker unit configured to amplify an audio signal from the controller 13 and emit sound. The sound emitted from the speaker 25 is a word or a call of the robot 100, and may be used to express the emotion of the robot 100 or the like.

The right-cheek light 26a and the left-cheek light 26b are examples of a light emitter provided on the head 2 of the robot 100. The right-cheek light 26a and the left-cheek light 26b are light modules configured to change at least one of a blinking speed, luminance, or a light color. The right-cheek light 26a and the left-cheek light 26b are configured of, for example, light emitting diode (LED) light modules. The robot 100 can guide breathing for the user by changing at least one of the blinking speed, the luminance, or the light color of the right-cheek light 26a and left-cheek light 26b.

The battery 15 is a power supply configured to supply power to each of the camera 11, the tactile sensor 12, the controller 13, the vital sensor 14, the first capacitive sensor 21, the second capacitive sensor 31, the right arm servo motor 35a, and the left arm servo motor 35b. Additionally, the battery 15 supplies power to each of the head servo motor 35c, the right leg servo motor 35d, and the left leg servo motor 35e. Further, the battery 15 supplies power to each of the right-eye display 24a, the left-eye display 24b, the speaker 25, the right-cheek light 26a, and the left-cheek light 26b. Various secondary batteries, such as a lithium ion battery and a lithium polymer battery can be used as the battery 15.

Here, installation positions of various sensors, such as the tactile sensor 12, the first capacitive sensor 21, and the second capacitive sensor 31 in the robot 100 can be appropriately changed. Additionally, various sensors, such as the tactile sensor 12, the first capacitive sensor 21, and the second capacitive sensor 31 may be disposed outside the robot 100 and may transmit necessary information to the robot 100 or an external device wirelessly.

Additionally, the robot 100 does not necessarily include the controller 13 inside the exterior member 10, and the controller 13 can communicate with each device from the outside of the exterior member 10 via wireless connection. The battery 15 can supply power to each component from the outside of the exterior member 10.

In the present embodiment, a configuration in which the head 2, the arm 3, and the leg 4 are displaceable is described as an example, but the present embodiment is not limited thereto, and at least one of the head 2, the arm 3, or the leg 4 may be displaceable. Additionally, the arm 3 is configured by a four-axis multi-joint robot arm, but may be configured by a six-axis multi-joint robot arm. Further, the arm 3 is preferably connectable to an end effector, such as a hand. Additionally, the leg 4 is configured by a wheel system, but can be configured by a crawler system, a leg system, or the like.

The configuration and shape of the robot 100 are not limited to those examples described in the present embodiment, and can be appropriately changed according to the preference of the user, the use form of the robot 100, and the like. For example, the robot 100 may be, instead of a form of imitating a bear cub, in a form of another living being, a form of a human, such as a humanoid, or the like. Additionally, the robot 100 may be in a form of a mobile device, such as a drone or a vehicle, including at least one of an arm, a display, a speaker, a light, or the like.

### < Example of Configuration of Camera 11 >

FIG. 4 is a view illustrating an example of the configuration of the camera 11. The camera 11 includes an imaging light source 201, a wavelength filter 202, a lens 203, and an imaging element 204. The camera 11 is disposed near a surface of the robot 100 while being camouflaged so as not to be visually recognized from the outside of the robot 100.

The imaging light source 201 irradiates a user 200 with irradiation light L having a predetermined peak wavelength. Although the predetermined peak wavelength is not particularly limited, the predetermined peak wavelength is preferably non-visible light, such as near-infrared light from the perspective of making the irradiation light less visible. The wavelength filter 202 is an optical element configured to transmit light having a wavelength near the peak wavelength in the irradiation light L from the imaging light source 201. The lens 203 forms an image of the user 200 or the like on an imaging surface of the imaging element 204 by using reflected light R, by the user 200 or the like, of the irradiation light L from the imaging light source 201. The imaging element 204 outputs, to the controller 13, a captured image Im obtained by capturing the image formed by the lens 203. The controller 13 can acquire respiratory property information of the user 200 based on the captured image Im. The respiratory property information includes information indicating a respiratory property, information related to a respiratory property, and the like. From the perspective that the respiratory property information can be acquired from the captured image Im, the captured image Im output from the imaging element 204 corresponds to the information on the breathing of the user. The imaging element may be a charge coupled device (CCD), a complementary metal-oxide semiconductor (CMOS), or the like. The captured image may be either a still image or a moving image.

In the camera 11, the wavelength filter 202 is disposed to camouflage the lens 203 and the imaging element 204 so that they are not easily visible from the outside of the robot 100. The camera 11 captures an image of the user 200, using the light reflected by the user 200 and transmitted through the wavelength filter 202 among the light from the imaging light source 201. Additionally, the nose 5 is a portion that the user 200 has fewer opportunities to touch than the trunk 1, the head 2, the arm 3, or the like. Thus, by disposing the camera 11 in the nose 5, the user 200 is less likely to contact the camera 11. Therefore, an uncomfortable feeling, caused by the surface of the camera 11 being harder than the surface of the exterior member 10 when the user 200 contacts the robot 100, can be reduced. Even when the user 200 contacts the nose 5, if the tactile feeling of the nose 5 is different from that of the trunk 1, the head 2, the arm 3, or the like, it is not so unnatural, and an uncomfortable feeling of the touch can be reduced.

The position where the camera 11 is disposed is not limited to the nose 5, but may be another portion, such as the mouth, the eye, or the like, as long as the above-described same effect as that of the arrangement on the nose 5 is obtained. Additionally, the camera 11 is not limited to be disposed inside the exterior member 10, but may be disposed outside the exterior member 10. When the camera 11 is disposed outside the exterior member 10, it is not necessarily required to camouflage the camera 11 to be less visible, and therefore the camera 11 does not need to include the wavelength filter 202 for camouflage.

In the present embodiment, the captured image Im by the camera 11 may be used not only for acquiring the respiration property information, but also for other purposes such as personal authentication of the user 200. Multiple cameras may be provided at multiple portions of the robot 100 for respective purposes.

The first detector is not limited to the camera 11, but may be an electromagnetic wave sensor, such as a Doppler sensor using electromagnetic waves, such as microwaves and millimeters. That is, in the present embodiment, the first detector may include at least one of an electromagnetic wave sensor configured to acquire the information on the breathing of the user, using electromagnetic waves, or an image sensor configured to acquire the information on the breathing of the user based on the captured image of the user.

For example, the microwave Doppler sensor as the electromagnetic wave sensor can detect vibrations on a body surface of the user 200 associated with breathing, and output information on the breathing of the user 200 by performing signal processing based on the detected vibrations. The vibration period output from the microwave Doppler sensor corresponds to the respiratory rate, frequency, or the like. The number of vibrations output from the microwave Doppler sensor corresponds to the respiratory count. The vibration amplitude output from the microwave Doppler sensor corresponds to the respiratory depth. To the microwave Doppler sensor, the vital sensor 14 described below with reference to FIG. 5 can be applied. The electromagnetic wave sensor does not use the captured image, and thus it is easier to dispose it inside the exterior member 10 in comparison with the camera 11. By disposing the first detector inside the exterior member 10, the user cannot visually recognize the first detector. As a result, the robot 100 reduces the resistance of the user to the acquisition of the information on the user's own breathing, and the information on the breathing of the user can be smoothly acquired.

The camera 11 may be used as the second detector to acquire information on at least one of the pulse, the heartbeat, the blood pressure, or the pulse pressure of the user. In this case, the irradiation light L and the reflected light R correspond to electromagnetic waves used to acquire biological information.

### <Configuration Example of Vital Sensor 14>

FIG. 5 is a diagram illustrating a configuration of the vital sensor 14. The vital sensor 14 is a microwave Doppler sensor including a microwave transmitter 141 and a microwave receiver 142. The microwave is an example of the electromagnetic wave.

The vital sensor 14 transmits a transmitted wave Ms, which is a microwave, toward the user 200 from the inside of the exterior member 10 by the microwave transmitter 141. The vital sensor 14 receives, by the microwave receiver 142, a reflected wave Mr of the transmitted wave Ms that is reflected by the user 200.

The vital sensor 14 detects, in a non-contact manner, a minute displacement generated on a body surface due to the beating of the heart of the user 200 or the like, based on a difference between the frequency of the transmitted wave Ms and the frequency of the reflected wave Mr, by using the Doppler effect. The vital sensor 14 can acquire information, such as a heartbeat, respiration, a pulse wave, a blood pressure, or a pulse pressure, as biological information of the user 200 based on the detected minute displacement, and output them to the controller 13. The information on the breathing includes the respiratory count, rhythm, respiratory depth, and the like. The pulse wave includes a pulse, a pulse interval R-R, a pulse wave waveform, a pulse wave propagation speed, and the like.

The vital sensor 14 is not limited to the microwave Doppler sensor, and may be a sensor configured to detect a minute displacement generated on a body surface by using a change in coupling between a human body and an antenna, or may be a sensor configured to use an electromagnetic wave other than the microwave, such as near-infrared light. Additionally, the vital sensor 14 may be a millimeter wave radar, a microwave radar, or the like. Further, the vital sensor 14 preferably includes a non-contact thermometer configured to detect infrared rays or the like transmitted from the user 200 in addition to the Doppler sensor. In this case, the vital sensor 14 detects biological information of the user 200 including information on at least one of a heartbeat (a pulse), respiration, a blood pressure, or a body temperature. Additionally, the vital sensor 14 may include multiple vital sensors respectively configured to acquire multiple types of biological information, such as the heartbeat, the respiration, the pulse wave, the blood pressure, and the pulse pressure, and acquire multiple types of biological information.

The vital sensor 14 is provided inside the exterior member 10, and thus the user 200 cannot visually recognize the vital sensor 14. This reduces resistance of the user 200 to the acquisition of the biological information, and enables the biological information to be smoothly acquired. Additionally, the vital sensor 14 can acquire the biological information in a non-contact manner, and thus can acquire the biological information even when the user 200 moves to some extent, unlike a contact sensor that requires the user 200 to be in contact with the same place for a certain period of time.

Additionally, by promoting the interaction between the user 200 and the robot 100 by the hug operation of the robot 100 or the like, the robot 100 is held by the user 200 and can acquire the biological information in a state of being in contact with or in proximity to the user 200. With this, the robot 100 can acquire highly reliable biological information in which noise is reduced.

### <Example of Configuration of Expansion-Contract Mechanism 18>

FIG. 6 is a view illustrating the configuration of the expansion-contraction mechanism 18. The expansion-contraction mechanism 18 includes a support 181, a press drive section 182, a rotation section 183, and a press section 184. The support 181 is fixed to the trunk frame 16 by a screw member, an adhesive member, or the like. The support 181 supports the press drive section 182.

The expansion-contraction mechanism 18 causes the press section 184 to oscillate back and forth around the rotational axis of the rotation section 183 by rotating the rotation section 183 around its rotation axis (in the directions of the arrow 180) by the press drive section 182. The expansion-contraction mechanism 18 can push or not push the exterior member 10 of the abdomen 191 by the oscillation of the press section 184. In the expansion-contraction mechanism 18, rotating the rotation section 183 clockwise around its rotation axis leads to a state in which the press section 184 pushes the exterior member 10 of the abdomen 191 from the inside to the outside. In this state, the exterior member 10 expands in the direction in which it is pushed by the press section 184, and the abdomen 191 expands. Conversely, rotating the rotation section 183 counterclockwise around its rotation axis leads to a state in which the press section 184 does not contact the exterior member 10 of the abdomen 191 and does not push the exterior member 10. In this state, the exterior member 10 contracts by its own elasticity, and the abdomen 191 contracts. The expansion-contraction mechanism 18 can expand and contract the abdomen 191 at a predetermined expansion-contraction frequency and a predetermined expansion-contraction amplitude in accordance with an expansion-contraction control signal from the controller 13. The abdomen 191 is a part of the trunk 1, and thus, in other words, the expansion-contraction mechanism 18 can expand and contract the trunk 1 at a predetermined expansion-contraction frequency and a predetermined expansion-contraction amplitude in accordance with the expansion-contraction control signal from the controller 13.

### <Modified Example of Expansion-Contraction Mechanism 18>

In order to reduce the energy consumption required for the expansion and contraction operation, the exterior member 10 at the position contacting the press section 184 may be formed with a cut or may be configured as an independent portion separated from the surroundings. In this case, if it is desired to add a "lifelike quality" to the robot 100, it is preferable to provide a cover member that hides the cut, on the surface. Additionally, the contact between the press section 184 and the exterior member 10 may be bonded. Additionally, as the expansion-contraction mechanism, a bag-like container may be disposed inside the exterior member 10, and may be controlled to be contracted by applying pressure to hydraulic pressure, air pressure, or another filled fluid. Additionally, as another expansion-contraction mechanism, a material, such as a soft actuator, which is deformed/expanded and contracted by applying an electric current may be disposed inside the exterior member 10, and the abdomen of the robot 100 may be expanded and contracted.

### <Configuration Example of Controller 13>

### (Hardware Configuration Example)

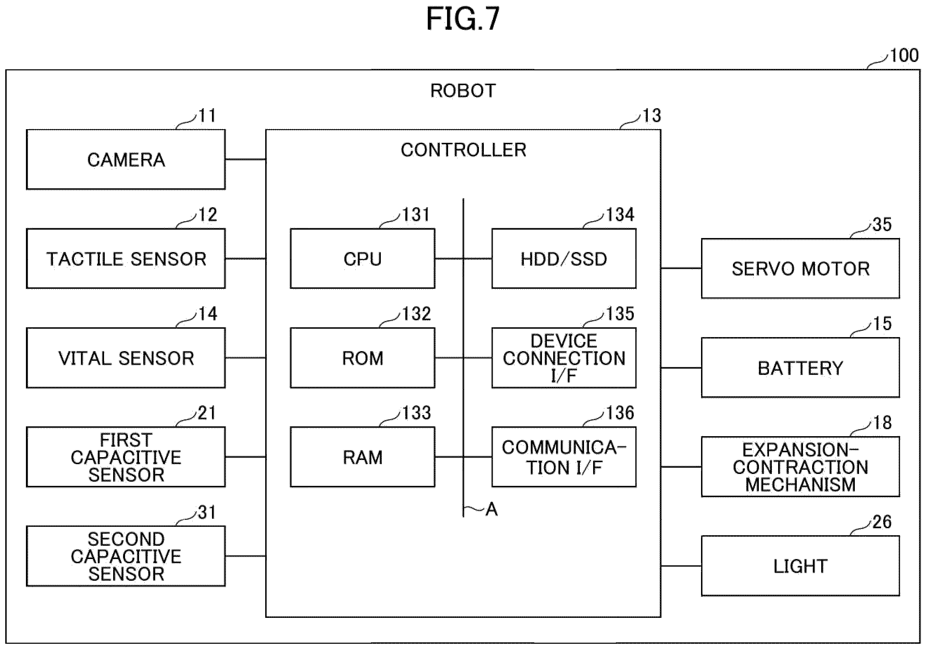

FIG. 7 is a block diagram illustrating an example of a hardware configuration of the controller 13. The controller 13 is constructed by a computer, and includes a central processing unit (CPU) 131, a read only memory (ROM) 132, and a random access memory (RAM) 133. Additionally, the controller 13 includes a hard disk drive/solid state drive (HDD/SSD) 134, a device connection interface (I/F) 135, and a communication I/F 136. These are communicably connected to each other via a system bus A.

The CPU 131 executes control processing including various arithmetic processing. The ROM 132 stores a program used to drive the CPU 131, such as an initial program loader (IPL). The RAM 133 is used as a work area of the CPU 131. The HDD/SSD 134 stores various information, such as programs, captured images acquired by the camera 11, detection information by various sensors, such as the biological information acquired by the vital sensor 14, the tactile signal acquired by the tactile sensor 12, and the like.

The device connection I/F 135 is an interface for connecting the controller 13 to various external devices. The external devices herein are the camera 11, the tactile sensor 12, the vital sensor 14, the first capacitive sensor 21, the second capacitive sensor 31, a servo motor 35, the battery 15, the expansion-contraction mechanism 18, the light 26, and the like. Additionally, the external devices include the display 24, the speaker 25, and the like as illustrated in FIG. 1.

Here, the servo motor 35 is a generic term of the right arm servo motor 35a, the left arm servo motor 35b, the head servo motor 35c, the right leg servo motor 35d, and the left leg servo motor 35e. Additionally, the display 24 is a generic term for the right-eye display 24a and the left-eye display 24b. Further, the light 26 is a generic term for the right-cheek light 26a and the left-cheek light 26b.

The communication I/F 136 is an interface for communicating with an external device via a communication network or the like. For example, the controller 13 is connected to the Internet via the communication I/F 136 and communicates with the external device via the Internet.

Here, at least some of the functions realized by the CPU 131 may be realized by an electric circuit or an electronic circuit.

### (Functional Configuration Example)

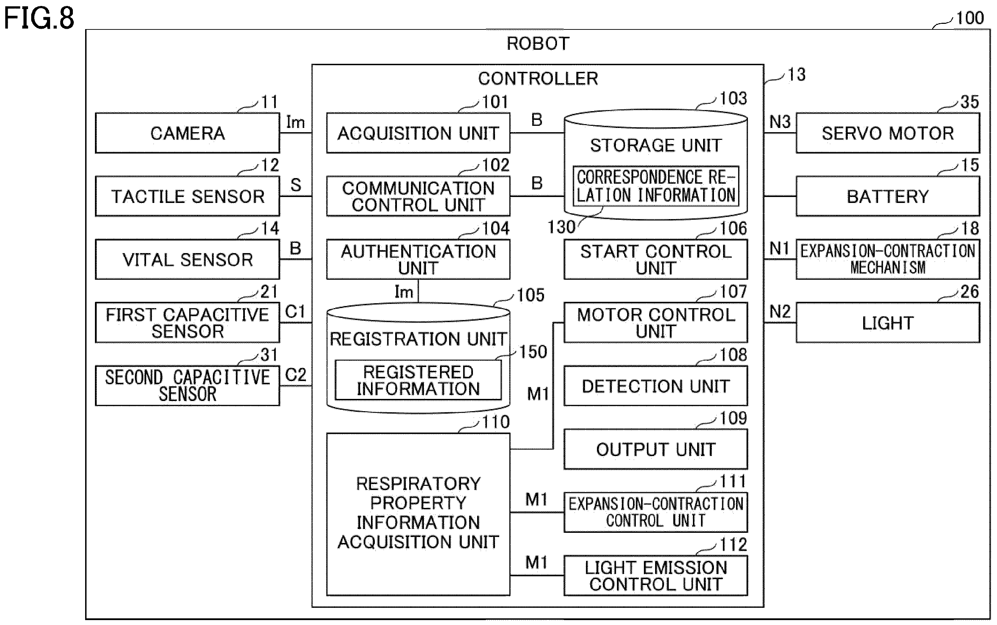

FIG. 8 is a block diagram illustrating an example of a functional configuration of the controller 13. The controller 13 includes an acquisition unit 101, a communication control unit 102, a storage unit 103, an authentication unit 104, a registration unit 105, a start control unit 106, a motor control unit 107, a detection unit 108, and an output unit 109. Further, the controller 13 includes a respiratory property information acquisition unit 110, an expansion-contraction control unit 111, and a light emission control unit 112. Here, the controller 13 may further include a function unit other than the above-described units.

The controller 13 can realize the functions of the acquisition unit 101 and the output unit 109 by the device connection I/F 135 or the like, and can realize the function of the communication control unit 102 by the communication I/F 136 or the like. Additionally, the controller 13 can realize the functions of the storage unit 103 and the registration unit 105 by a non-volatile memory, such as the HDD/SSD 134. Further, the controller 13 can realize the functions of the authentication unit 104, the start control unit 106, the motor control unit 107, and the detection unit 108 by a processor, such as the CPU 131, executing processing defined in a program stored in a non-volatile memory, such as the ROM 132, or the like.

Additionally, the controller 13 can realize the functions of the respiratory property information acquisition unit 110, the expansion-contraction control unit 111, and the light emission control unit 112 by a processor, such as the CPU 131, executing processing defined in a program stored in a non-volatile memory, such as the ROM 132, or the like. Here, some of the above-described functions of the controller 13 may be realized by an external device, such as a PC or a server, or may be realized by distributed processing between the controller 13 and the external device.

The acquisition unit 101 controls communication between the controller 13 and the camera 11 to acquire the captured image Im of the user 200 from the camera 11. Additionally, the acquisition unit 101 controls communication between the controller 13 and the tactile sensor 12 to acquire a tactile signal S from the tactile sensor 12. Further, the acquisition unit 101 controls communication between the controller 13 and the vital sensor 14 to acquire biological information B of the user 200 from the vital sensor 14.

Additionally, the acquisition unit 101 controls communication between the controller 13 and the first capacitive sensor 21 to acquire a first capacitance signal C1 from the first capacitive sensor 21. Additionally, the acquisition unit 101 controls communication between the controller 13 and the second capacitive sensor 31 to acquire a second capacitance signal C2 from the second capacitive sensor 31.

The communication control unit 102 controls communication with an external device via a communication network or the like. For example, the communication control unit 102 can transmit the captured image Im acquired by the camera 11, the biological information B acquired by the vital sensor 14, the tactile signal S acquired by the tactile sensor 12, and the like to the external device via the communication network.

The storage unit 103 stores the biological information B acquired by the vital sensor 14. The storage unit 103 continuously stores the acquired biological information B while the acquisition unit 101 is acquiring the biological information B from the vital sensor 14. Additionally, the storage unit 103 can store information obtained from the captured image Im by the camera 11, the tactile signal S from the tactile sensor 12, the first capacitance signal C1 from the first capacitive sensor 21, and the second capacitance signal C2 from the second capacitive sensor 31. Additionally, the storage unit 103 may store predetermined correspondence relation information with respect to the correspondence relation between the respiratory property information acquired based on the captured image Im and at least one of the operation of the expansion-contraction mechanism 18, the operation of the light 26, or the servo motor 35.

The authentication unit 104 performs personal authentication of the user 200 based on the captured image Im of the user 200 captured by the camera 11. For example, the authentication unit 104 performs face authentication by referring to registered information 150 of a face image registered in advance in the registration unit 105, based on the captured image Im including the face of the user 200 captured by the camera 11. With this, the user 200 currently in contact with or in proximity to the robot 100 can be associated with the personal information registered in advance, and the biological information B acquired by the vital sensor 14 can be associated with the personal information. Additionally, the controller 13 can also perform control so as to stop the start of the acquisition of the biological information by the vital sensor 14 when the face image included in the captured image Im is not registered in the registration unit 105.

The start control unit 106 causes the vital sensor 14 to start acquiring the biological information B. For example, when the contact or proximity of the user 200 with respect to the robot 100 is detected by the detection unit 108, the start control unit 106 turns on a switch or the like for supplying power from the battery 15 to the vital sensor 14. With this, the start control unit 106 causes the vital sensor 14 to start acquiring the biological information B.

The detection unit 108 detects the contact or proximity of the user 200 with respect to the robot 100 based on the captured image Im and the like obtained by the camera 11. The detection unit 108 may detect the distance from the robot 100 to the user 200 based on the captured image Im obtained by the camera 11. Additionally, the detection unit 108 may detect the contact or proximity of the user 200 with respect to the robot 100 based on the first capacitance signal C1 or the second capacitance signal C2. Further, the detection unit 108 may detect the contact or proximity of the user 200 with respect to the robot 100 based on the tactile signal S from the tactile sensor 12.

The respiratory property information acquisition unit 110 acquires respiratory property information M1 of the user 200 (see FIG. 4) included in the captured image Im based on the captured image Im acquired via the acquisition unit 101. For example, the respiratory property information acquisition unit 110 detects the vibration of the chest of the user 200 associated with breathing from multiple captured images Im continuously acquired via the acquisition unit 101. The respiratory property information acquisition unit 110 acquires the respiratory property information M1, such as the respiratory speed, frequency, the respiratory count, and the respiratory depth from the vibration. The respiratory property information acquisition unit 110 may acquire the respiratory property information M1 by remote photoplethysmography (rPPG) based on the captured image Im. rPPG is a technology for estimating the heartbeat and respiration by analyzing a change in a skin color caused by blood flow. The respiratory property information acquisition unit 110 may acquire the respiratory property information M1 based on the biological information B acquired using the vital sensor 14. The respiratory property information acquisition unit 110 outputs the respiratory property information M1 to the expansion-contraction control unit 111.

The expansion-contraction control unit 111 controls the operation of the expansion-contraction mechanism 18 so as to guide the breathing of the user 200 to a predetermined state in accordance with the respiratory property information M1 from the respiratory property information acquisition unit 110. For example, based on the respiratory property information M1, the expansion-contraction control unit 111 acquires operation information N1 of the expansion-contraction mechanism 18 by referring to correspondence relation information 130 stored in the storage unit 103. The expansion-contraction control unit 111 can control the operation of the expansion-contraction mechanism 18 by outputting the operation information N1 to the expansion-contraction mechanism 18 via the output unit 109. The operation information N1 corresponds to the expansion-contraction control signal.

The Table 1 below indicates an example of the correspondence relation information 130. In Table 1, the correspondence relation information 130 includes the respiratory rates V1-V4 and the respiratory depths D1-D4 as the respiratory property information M1. Additionally, the correspondence relation information 130 includes the expansion-contraction frequencies f1-f4 paired with the respiratory rates V1-V4 and the expansion-contraction amplitudes Am1-Am4 paired with the respiratory depths D1-D4.

**[Table 1]**

| No | M1 | N1 |
|---|---|---|
| 1 | RESPIRATORY RATE V1 | EXPANSION-CONTRACTION FREQUENCY f1 |
| 2 | RESPIRATORY RATE V2 | EXPANSION-CONTRACTION FREQUENCY f2 |
| 3 | RESPIRATORY RATE V3 | EXPANSION-CONTRACTION FREQUENCY f3 |
| 4 | RESPIRATORY RATE V4 | EXPANSION-CONTRACTION FREQUENCY f4 |
| 5 | RESPIRATORY DEPTH D1 | EXPANSION-CONTRACTION AMPLITUDE Am1 |
| 6 | RESPIRATORY DEPTH D2 | EXPANSION-CONTRACTION AMPLITUDE Am2 |
| 7 | RESPIRATORY DEPTH D3 | EXPANSION-CONTRACTION AMPLITUDE Am3 |
| 8 | RESPIRATORY DEPTH D4 | EXPANSION-CONTRACTION AMPLITUDE Am4 |
| • | • | • |

For example, the expansion-contraction frequency in the operation information N1 is predetermined as a frequency shifted by a predetermined frequency from the frequency corresponding to the respiratory rate in the respiratory property information M1. The expansion-contraction control unit 111 acquires information on an expansion-contraction frequency that is shifted by a predetermined frequency from the frequency corresponding to the respiratory rate in the respiratory property information M1 by referring to the correspondence relation information 130. The expansion-contraction control unit 111 expands and contracts the expansion-contraction mechanism 18 with the expansion-contraction frequency. The user 200 breathes in accordance with the expansion and contraction of the expansion-contraction mechanism 18. With this, the robot 100 can guide breathing for the user 200 so that the respiratory rate gradually slows, for example. Each of states in a time series in which the respiratory rate gradually slows corresponds to a "predetermined state" of the breathing.

Additionally, for example, the expansion-contraction amplitude in the operation information N1 is predetermined as an amplitude shifted by a predetermined amplitude from the amplitude corresponding to the respiratory depth in the respiratory property information M1. The expansion-contraction control unit 111 acquires information on an expansion-contraction amplitude that is shifted by a predetermined amplitude from the amplitude corresponding to the respiratory depth in the respiratory property information M1 by referring to the correspondence relation information 130. The expansion-contraction control unit 111 expands and contracts the expansion-contraction mechanism 18 with the expansion-contraction amplitude. The user 200 breathes in accordance with the expansion and contraction of the expansion-contraction mechanism 18. With this, the robot 100 can guide breathing for the user 200 by increasing the expansion-contraction amplitude so that the respiratory depth gradually increases, for example. Each of states in which the respiratory depth gradually increases corresponds to a "predetermined state" of the breathing.

The operation of the expansion-contraction mechanism 18 is an expansion-contraction movement of the trunk 1 of the robot 100 and corresponds to the operation of the robot 100. Therefore, the robot 100 can guide breathing for the user 200 by the operation of the robot 100 controlled by the expansion-contraction control unit 111 in a state where the user 200 holds the robot 100, interacts with the robot 100, and communicates with the robot 100. Here, the correspondence relation information 130 indicated in Table 1 is an example and the embodiment is not limited thereto. Additionally, the method of guiding the breathing by the expansion-contraction control unit 111 is an example and the embodiment is not limited thereto. For example, the robot 100 may estimate the operation of the expansion-contraction mechanism 18 for properly guiding the breathing by using a deep neural network (DNN) or the like based on the respiratory property information M1, and control the operation of the expansion-contraction mechanism 18 in accordance with the estimation result.

The respiratory property information acquisition unit 110 may output the respiratory property information M1 to the light emission control unit 112. The light emission control unit 112 may control the operation of the light 26 so as to guide the breathing of the user 200 to a predetermined state in accordance with the respiratory property information M1 from the respiratory property information acquisition unit 110. The light emission control unit 112 may control at least one of the blinking speed of the light 26, the luminance of the light from the light 26, or the color of the light from the light 26. For example, the light emission control unit 112 acquires operation information N2 of the light 26 by referring to the correspondence relation information stored in the storage unit 103 based on the respiratory property information M1. The operation information N2 of the light 26 is information for controlling at least one of the blinking speed of the light 26, the luminance of the light from the light 26, or the color of the light from the light 26. The light emission control unit 112 can control the operation of the light 26 by outputting the operation information N2 of the light 26 to the light 26 via the output unit 109. The user 200 breathes in accordance with the blinking speed of the light 26 with visually recognizing the light 26 in a state of holding the robot 100, interacting with the robot 100, and communicating with the robot 100. With this, the robot 100 can guide breathing for the user 200.

The respiratory property information acquisition unit 110 may output the respiratory property information M1 to the motor control unit 107. The motor control unit 107 may control the operation of the servo motor 35 so as to guide the breathing of the user 200 to a predetermined state in accordance with the respiratory property information M1 from the respiratory property information acquisition unit 110. By controlling the operation of the servo motor 35, the motor control unit 107 can control the drive section including the arm 3 connected to the robot main body in the robot 100 so as to be relatively displaceable. The motor control unit 107 may control at least one of an operation of wrapping the arm 3 around a part of the body of the user 200, an operation of pressurizing the skin of the user 200 by the arm 3, or an operation of stroking a part of the body of the user 200 by the arm 3. For example, the motor control unit 107 acquires operation information N3 of the servo motor 35 by referring to the correspondence relation information stored in the storage unit 103 based on the respiratory property information M1. The motor control unit 107 can control the operation of the servo motor 35 by outputting the operation information N3 of the servo motor 35 to the servo motor 35 via the output unit 109. The user 200 breathes in sync with the movement of the arm 3 in a state of holding the robot 100, interacting with the robot 100, and communicating with the robot 100. With this, the robot 100 can guide breathing for the user 200.

### <Example of Operation of Robot 100>

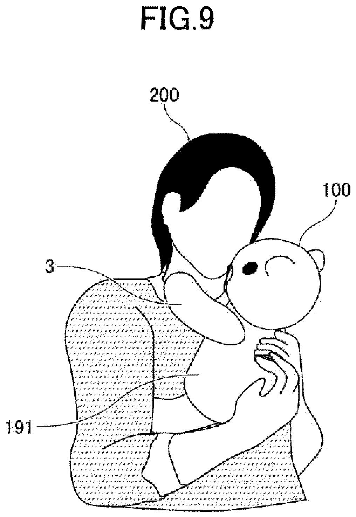

FIG. 9 is a view illustrating a state of the breathing guide by the robot 100. In FIG. 9, the user 200 holds the robot 100, interacts with the robot 100, and communicates with the robot 100. In this state, the robot 100 expands and contracts the abdomen 191 by the expansion-contraction control unit 111 illustrated in FIG. 8. The robot 100 makes the user 200 holding the robot 100 feel the expansion and contraction of the abdomen 191. The user 200 breathes in sync with the expansion and contraction of the abdomen 191. As described, the robot 100 can guide breathing for the user 200.

Alternatively, in the state of FIG. 9, the robot 100 controls at least one of an operation of wrapping the arm 3 around a part of the body of the user 200, an operation of pressing the skin of the user 200 by the arm 3, or an operation of stroking a part of the body of the user 200 by the arm 3, by the motor control unit 107 illustrated in FIG. 9. With this, the robot 100 can guide breathing for the user 200 in sync with the movement of the arm 3 by making the user 200 holding the robot 100 feel the movement of the arm 3. In addition to the above, the robot 100 may guide breathing for the user 200 by controlling at least one of the blinking speed of the light 26, the luminance of the light from the light 26, or the color of the light from the light 26, by the light emission control unit 112 illustrated in FIG. 9.

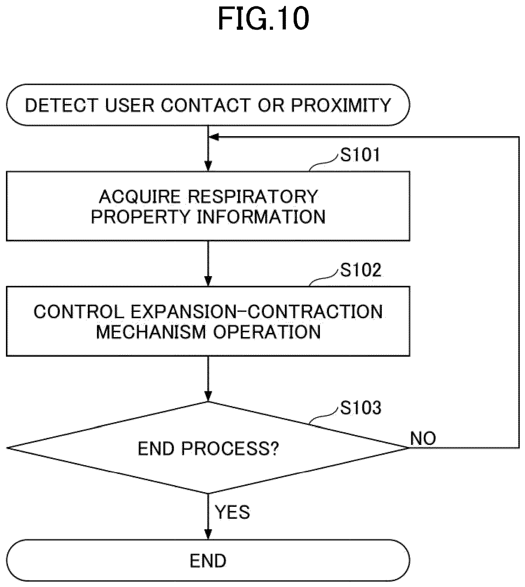

FIG. 10 is a flowchart illustrating a process by the controller 13. FIG. 10 illustrates an example of the process by the controller 13 for guiding breathing for the user 200. The controller 13 starts the process of FIG. 10 when the detection unit 108 detects the contact or proximity of the user 200 to the robot 100. Hereinafter, the description will be provided with reference to the functional configuration diagram of FIG. 8 as appropriate.

First, in step S101, the controller 13 acquires the respiratory property information M1 of the user 200 included in the captured image Im by the respiratory property information acquisition unit 110 based on the captured image Im acquired via the acquisition unit 101. The respiratory property information acquisition unit 110 outputs the respiratory property information M1 to the expansion-contraction control unit 111.

Next, in step S102, the controller 13 controls, by the expansion-contraction control unit 111, the operation of the expansion-contraction mechanism 18 so as to guide the breathing of the user 200 to a predetermined state in accordance with the respiratory property information M1 from the respiratory property information acquisition unit 110.

Subsequently, in step S103, the controller 13 determines whether to end the process. For example, the controller 13 may determine to end the process if a predetermined time has elapsed, and may determine not to end the process if the predetermined time has not elapsed. Alternatively, the controller 13 may determine to end the process if it is detected that the user 200 is neither in contact with nor in proximity to the robot 100, and may determine not to end the process if it is detected that the user 200 is in contact with or in proximity to the robot 100. However, other determination methods may be used.

If it is determined in step S103 that the process is not to be ended (step S103, NO), the controller 13 performs the process from step S101 again. If it is determined in step S103 that the process is to be ended (step S103, YES), the controller 13 ends the process.

As described above, the controller 13 can perform the process for causing the robot 100 to guide breathing for the user 200. Here, although the process of controlling the operation of the expansion-contraction mechanism 18 is exemplified here, the flowchart of FIG. 10 can also be applied to the process of controlling the operation of the servo motor 35 or the light 26 by replacing the process by the expansion-contraction control unit 111 with the process by the motor control unit 107 or the light emission control unit 112.

### <Main Operation and Effect of Robot 100>

As described above, the robot 100 includes the exterior member 10, at least one of the camera 11 or the vital sensor 14 (the first detector) configured to acquire the information on the breathing of the user, and a controller 13 configured to control the operation of the robot 100 so as to guide the breathing of the user to the predetermined state based on the information acquired by the first detector. For example, the controller 13 guides the breathing of the user to the predetermined state by controlling an operation of at least one of the drive sections including the expansion-contraction mechanism 18, the light 26 (the light emitter), and the arm 3.

In the present embodiment, the robot 100 guides breathing for the user 200 in a state in which the robot 100 and the user 200 communicate with each other, and thus the robot 100 can naturally guide breathing for the user 200. In other words, in the present embodiment, a robot that can naturally guide breathing for the user can be provided.

Additionally, in the present embodiment, the robot 100 acquires the respiratory property information M1 of the user 200 in an unconstrained natural state in which the camera 11 does not contact the user 200 during a period in which the robot 100 and the user 200 interact with each other. The robot 100 controls its own operation based on the respiratory property information M1. Therefore, the robot 100 can naturally guide breathing for the user 200 without causing the user 200 to feel restrained.

Additionally, the robot 100 grasps the state of the user 200 by the first detector and guides the breathing in accordance with the state of the user 200, thereby realizing appropriate breathing guidance.

Additionally, in the present embodiment, the breathing is guided by the operation of the robot 100, and thus many opportunities of interaction between the user 200 and the robot 100 are easily created. With this, the robot 100 can guide breathing for the user 200 among many opportunities of the interaction.

### [Second Embodiment]

A robot according to a second embodiment will be described below. The present embodiment differs from the first embodiment in that the operation of the robot is controlled so as to guide the breathing of the user to a predetermined state additionally based on information on at least one of the pulse, the heartbeat, the blood pressure, and the pulse pressure of the user, obtained by the second detector, such as a vital sensor. Here, the same names and symbols as those of the first embodiment indicate the same or substantially the same members, and detailed description thereof will be omitted accordingly. This point also applies to the embodiments described below.

### <Example of Functional Configuration of Controller 13a>

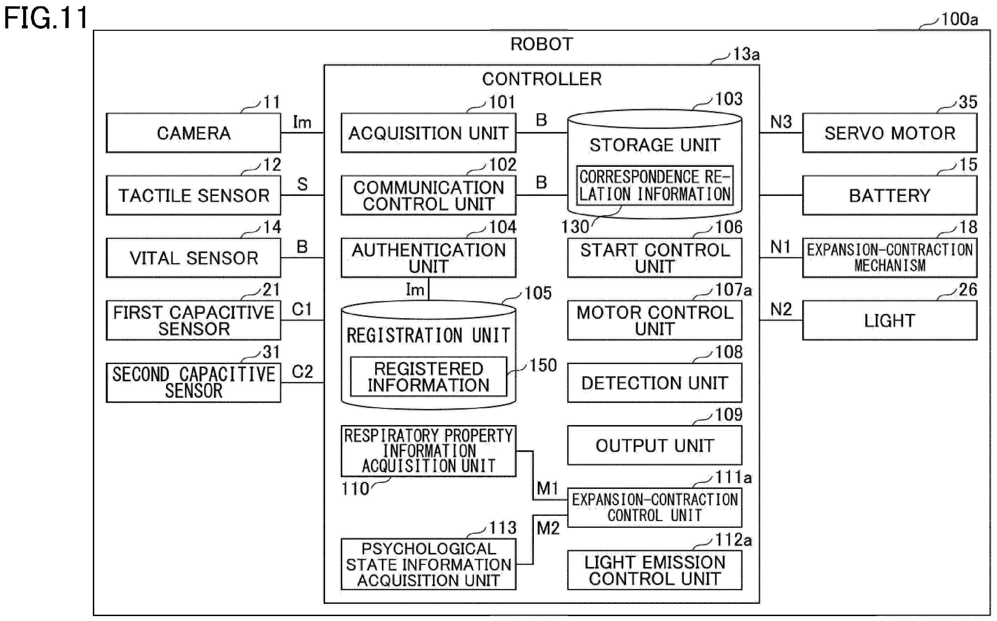

FIG. 11 is a block diagram illustrating a functional configuration of a controller 13a of a robot 100a according to the second embodiment. The controller 13a includes a psychological state information acquisition unit 113, an expansion-contraction control unit 111a, a motor control unit 107a, and a light emission control unit 112a.

The controller 13a can realize the functions of the psychological state information acquisition unit 113, the expansion-contraction control unit 111a, the motor control unit 107a, and the light emission control unit 112a by a processor, such as the CPU 131 illustrated in FIG. 7, executing processing defined by a program stored in a nonvolatile memory, such as the ROM 132, or the like. Here, a part of the above-described function of the controller 13a may be realized by an external device, such as a PC or a server, or may be realized by distributed processing between the controller 13 and the external device.

The psychological state information acquisition unit 113 acquires psychological state information M2 based on the information on at least one of the pulse, the heart rate, the blood pressure, or the pulse pressure of the user output from the vital sensor 14. The psychological state information M2 is information on a psychological state of the user 200. The psychological state information M2 includes information indicating the degree of relaxation of the user 200, information related to the degree of relaxation of the user 200, information indicating the degree of stress of the user 200, information related to the degree of stress of the user 200, information indicating the emotion of the user 200, information related to the emotion of the user 200, and the like. For example, as the user 200 relaxes, the pulse rate, the heart rate, and the like of the user 200 decrease, or the blood pressure, the pulse pressure, and the like decrease. Therefore, the psychological state information acquisition unit 113 can acquire the psychological state information M2 indicating the degree of relaxation of the user 200 and the like from the output of the vital sensor 14.

The psychological state information acquisition unit 113 outputs the acquired psychological state information M2 to the expansion-contraction control unit 111a. The expansion-contraction control unit 111a controls the operation of the expansion-contraction mechanism 18 so as to guide the breathing of the user 200 to a predetermined state based on the respiratory property information M1 and the psychological state information M2.

The psychological state information acquisition unit 113 may output the psychological state information M2 to at least one of the expansion-contraction control unit 111a, the light emission control unit 112a, or the motor control unit 107a. The light emission control unit 112a can control the operation of the light 26 so as to guide the breathing of the user 200 to a predetermined state based on the respiratory property information M1 and the psychological state information M2. The motor control unit 107a can control the operation of the servo motor 35 so as to guide the breathing of the user 200 to a predetermined state based on the respiratory property information M1 and the psychological state information M2.

### <Example of Process by Controller 13a>

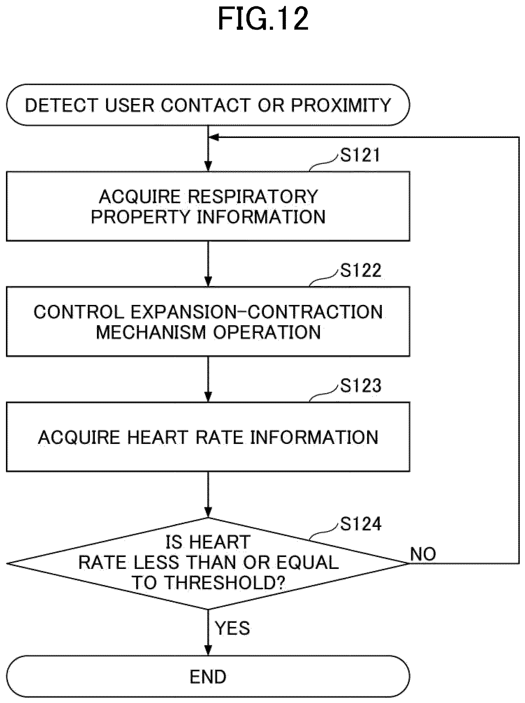

FIG. 12 is a flowchart illustrating a process by the controller 13a. FIG. 12 illustrates the process by the controller 13a to guide breathing for the user 200. When the detection unit 108 detects the contact or proximity of the user 200 to the robot 100a, the controller 13a starts the process of FIG. 12. Hereinafter, the description will be provided with reference to the functional configuration diagram of FIG. 11 as appropriate. Additionally, the processing of steps S121 to S122 in FIG. 12 is the same as the processing of steps S101 to S102 in FIG. 10, and an overlapping description will be omitted here.

In step S123, the controller 13a acquires, by the psychological state information acquisition unit 113, heart rate information as the psychological state information M2 of the user 200 based on the information on the heart rate of the user 200 output from the vital sensor 14. The psychological state information acquisition unit 113 outputs the acquired heart rate information to the expansion-contraction control unit 111a.

Subsequently, in step S124, the expansion-contraction control unit 111a determines whether the heart rate in the heart rate information acquired from the psychological state information acquisition unit 113 is less than or equal to a threshold. For example, the threshold of the heart rate is predetermined and stored in the storage unit 103. The expansion-contraction control unit 111a can acquire the threshold information on the heart rate by referring to the storage unit 103.

If it is determined in step S124 that the heart rate is not less than or equal to the threshold (step S124, NO), the controller 13a determines that the user 200 is not relaxed, and performs the processing as of step S121 again. If it is determined in step S124 that the heart rate is less than or equal to the threshold (step S124, YES), the controller 13a determines that the user 200 is relaxed, and ends the process.

As described above, the controller 13a can perform a process of guiding breathing for the user 200 based on the respiratory property information M1 of the user 200 and the heart rate information of the user 200. Here, the heart rate information is exemplified as the psychological state information M2, but the psychological state information M2 may be information on at least one of the pulse rate, the blood pressure, or the pulse pressure of the user. Additionally, although the process of controlling the operation of the expansion-contraction mechanism 18 is exemplified here, the flowchart of FIG. 12 can also be applied to the process of controlling the operation of the servo motor 35 or the light 26 by replacing the process by the expansion-contraction control unit 111a with the process by the motor control unit 107a or the light emission control unit 112a.

### <Main Operation Effect of Robot 100a>

As described above, in the present embodiment, the controller 13a controls the operation of the robot 100a so as to guide the breathing of the user to a predetermined state based on the information on the respiration of the user acquired by one of the camera 11 or the vital sensor 14 (the first detector) and the information on at least one of the pulse rate, the heart rate, the blood pressure, or the pulse pressure of the user acquired by the vital sensor 14 (the second detector). For example, the controller 13a controls the operation of the robot 100a so as to guide the breathing of the user to a predetermined state in accordance with the information on the psychological state of the user obtained based on the output from the vital sensor 14.

In the present embodiment, the psychological state of the user, such as the state of relaxation, can be grasped from the information on at least one of the pulse rate, the heart rate, the blood pressure, or the pulse pressure of the user, and thus the breathing of the user can be guided to improve the psychological state. For example, the robot 100a determines whether the user is in a relaxed state after performing the breathing guidance, and if not, it further performs the breathing guidance to the user 200 to lead the user 200 to the relaxed state. Here, other operation effects are the same as those of the first embodiment.

### [Third Embodiment]

A robot according to a third embodiment will be described below. The present embodiment differs from the first embodiment in that at least one of an abdomen expansion-contraction mechanism or a back expansion-contraction mechanism included in the expansion-contraction mechanism is caused to expand and contract based on information on the state of the robot when held by the user.

### <Configuration Example of Robot 100b>

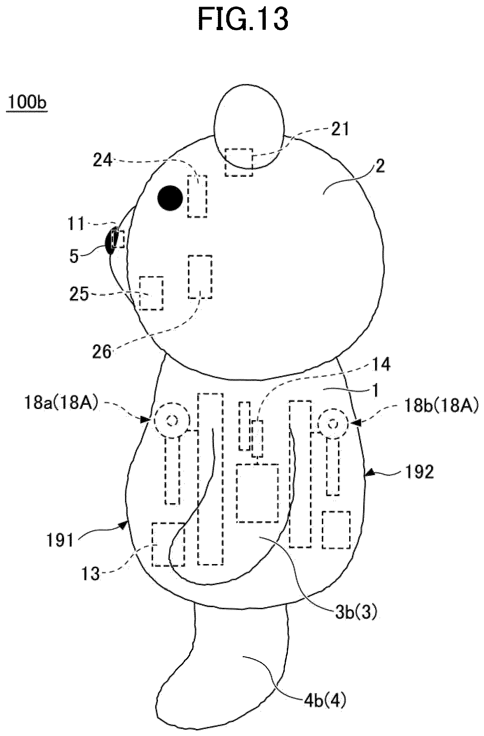

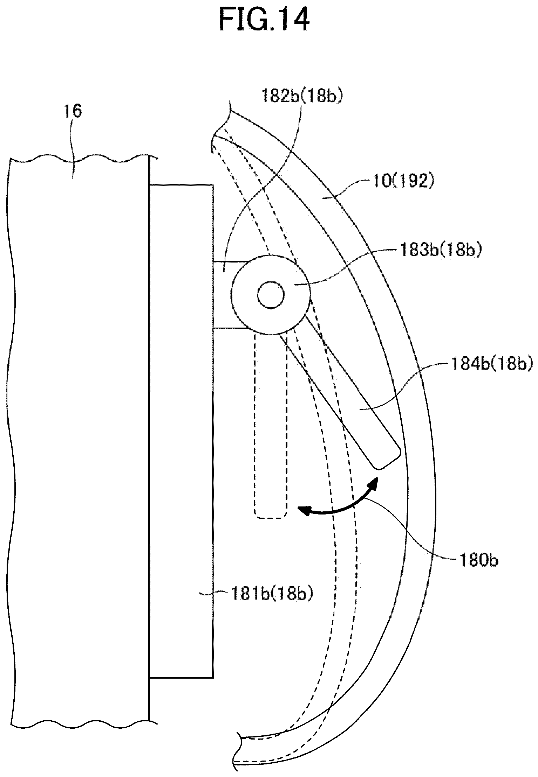

FIGS. 13 and 14 are views illustrating an expansion-contraction mechanism 18A in the robot 100b according to the third embodiment. FIG. 13 is a view illustrating an example of an arrangement of the expansion-contraction mechanism 18A in the robot 100b. FIG. 14 is a view illustrating an example of a configuration of a back expansion-contraction mechanism 18b in the expansion-contraction mechanism 18A.

As illustrated in FIG. 13, the expansion-contraction mechanism 18A includes an abdomen expansion-contraction mechanism 18a disposed in the abdomen 191 of the robot 100b and the back expansion-contraction mechanism 18b disposed in the back 192 of the robot 100b. The abdomen expansion-contraction mechanism 18a is disposed to be able to push the exterior member 10 of the abdomen 191. The configuration and function of the abdomen expansion-contraction mechanism 18a are the same as those of the above-described expansion-contraction mechanism 18, and thus an overlapping description will be omitted here.

The back expansion-contraction mechanism 18b is disposed to be able to push the exterior member 10 of the back 192. As illustrated in FIG. 14, the back expansion-contraction mechanism 18b includes a support 181b, a press drive section 182b, a rotation section 183b, and a press section 184b. The support 181b is fixed, by a screw member, an adhesive member, or the like, to the side of the trunk frame 16 that is opposite to the side to which the support 181 is fixed. That is, the support 181 of the abdomen expansion-contraction mechanism 18a is fixed to the abdomen 191 side of the trunk frame 16, and the support 181b of the back expansion-contraction mechanism 18b is fixed to the back 192 side of the trunk frame 16. The support 181b supports the press drive section 182b.

The back expansion-contraction mechanism 18b causes the press section 184b to oscillate back and forth around the rotational axis of the rotation section 183b by rotating the rotation section 183b around its rotation axis (in the directions of the arrow 180b) by the press drive section 182b. The back expansion-contraction mechanism 18b can push or not push the exterior member 10 of the back 192 by the oscillation of the press section 184b. In the back expansion-contraction mechanism 18b, rotating the rotation section 183b counterclockwise around its rotation axis leads to a state in which the press section 184b pushes the exterior member 10 of the back 192 from the inside to the outside. In this state, the exterior member 10 expands in the direction in which it is pushed by the press section 184b, and the back 192 expands. Conversely, rotating the rotation section 183b clockwise around its rotation axis leads to a state in which the press section 184b does not contact the exterior member 10 of the back 192 and does not push the exterior member 10. In this state, the exterior member 10 contracts by its own elasticity, and the back 192 contracts. The back expansion-contraction mechanism 18b can expand and contract the back 192 at a predetermined expansion-contraction frequency and a predetermined expansion-contraction amplitude in accordance with a back expansion-contraction control signal from a controller 13b.

### <Example of Functional Configuration of Controller 13b>

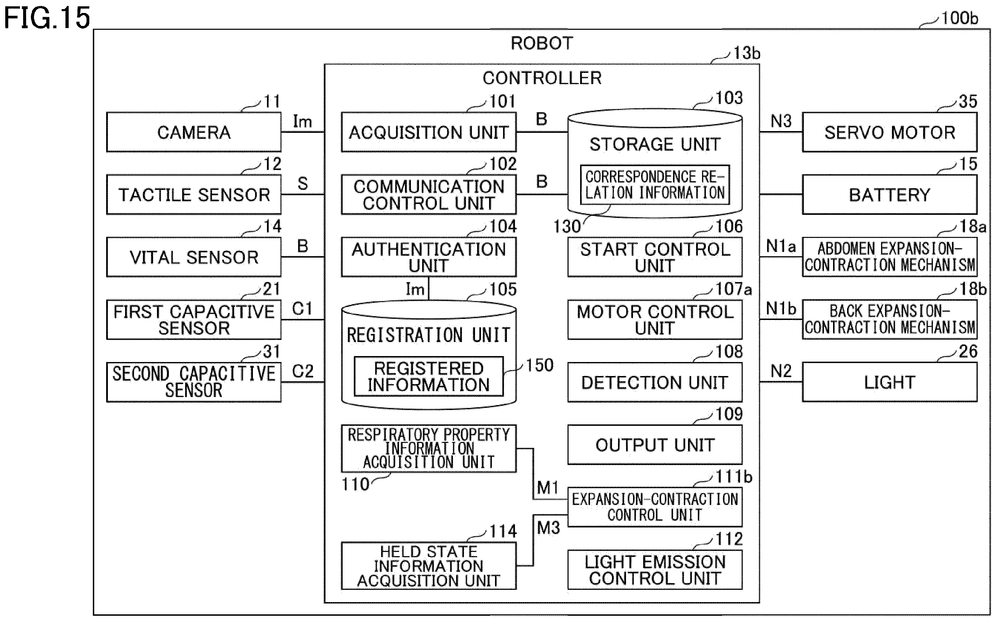

FIG. 15 is a block diagram illustrating a functional configuration of the controller 13b of the robot 100b. The controller 13b includes a held state information acquisition unit 114 and an expansion-contraction control unit 111b.

The controller 13b can realize the functions of the held state information acquisition unit 114 and the expansion-contraction control unit 111b by a processor, such as the CPU 131, illustrated in FIG. 7 executing processing defined in a program stored in a nonvolatile memory, such as the ROM 132, or the like. Here, a part of the functions of the controller 13b may be realized by an external device, such as a PC or a server, or may be realized by distributed processing between the controller 13b and the external device.

The held state information acquisition unit 114 acquires held state information M3, which is information on the state of the robot 100b when held by the user 200. For example, when the user 200 holds the robot 100b such that the abdomen 191 of the robot 100b is in contact with the user 200, the held state information acquisition unit 114 acquires the held state information M3 indicating that the abdomen 191 is in contact with the user 200. When the user 200 holds the robot 100b such that the back 192 of the robot 100b is in contact with the user 200, the held state information acquisition unit 114 acquires the held state information M3 indicating that the back 192 is in contact with the user 200. The held state information acquisition unit 114 can detect whether the abdomen 191 or the back 192 of the robot 100b is in contact with the user 200 by performing image processing on the captured image Im obtained by the camera 11, for example, and acquire the held state information M3.

The held state information acquisition unit 114 outputs the acquired held state information M3 to the expansion-contraction control unit 111b. The expansion-contraction control unit 111b controls the operation of the expansion-contraction mechanism 18A so as to guide the breathing of the user 200 to a predetermined state based on the respiratory property information M1 and the held state information M3. In other words, the expansion-contraction control unit 111b expands and contracts at least one of the abdomen expansion-contraction mechanism 18a or the back expansion-contraction mechanism 18b based on the held state information M3. Specifically, based on the held state information M3, the expansion-contraction control unit 111b controls the operation of the abdomen expansion-contraction mechanism 18a by outputting operation information N1a via the output unit 109 when the user 200 holds the robot 100b such that the abdomen 191 is in contact with the user 200. Additionally, based on the held state information M3, the expansion-contraction control unit 111b controls the operation of the back expansion-contraction mechanism 18b by outputting operation information N2a via the output unit 109 when the user 200 holds the robot 100b such that the back 192 is in contact with the user 200.

### <Example of Process by Controller 13b>

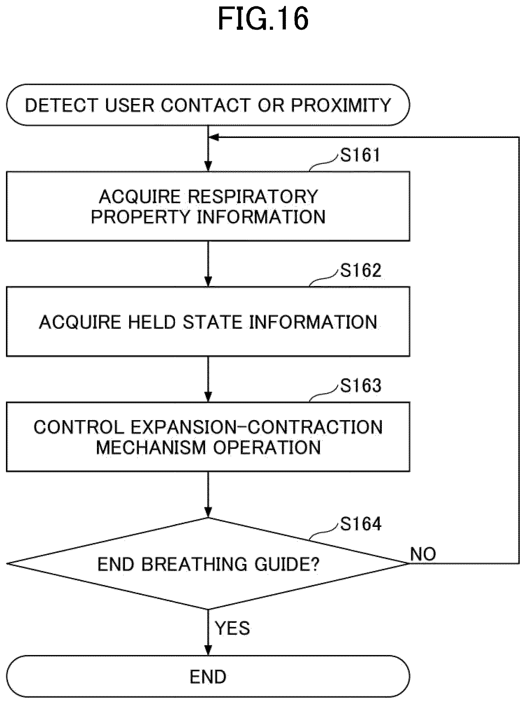

FIG. 16 is a flowchart illustrating a process by the controller 13b. FIG. 16 illustrates the process by the controller 13b to guide breathing for the user 200. The controller 13b starts the process illustrated in FIG. 16 when the detection unit 108 detects the contact or proximity of the user 200 to the robot 100b. Hereinafter, the description will be provided with reference to the functional configuration of FIG. 15 as appropriate. Additionally, the processing of step S161 in FIG. 16 is the same as that of step S101 in FIG. 10, and thus an overlapping description will be omitted here.

In step S162, the controller 13b acquires, by the held state information acquisition unit 114, the held state information M3, which is the information on the state of the robot 100b when held by the user 200. The held state information acquisition unit 114 outputs the acquired held state information M3 to the expansion-contraction control unit 111b.

Subsequently, in step S163, the controller 13b controls, by the expansion-contraction control unit 111b, the operation of the expansion-contraction mechanism 18A so as to guide the breathing of the user 200 to a predetermined state based on the respiratory property information M1 and the held state information M3.

Subsequently, in step S164, the controller 13b determines whether to end the process. For example, the controller 13b can determine to end the process if a predetermined time has elapsed, and not to end the process if the predetermined time has not elapsed. Alternatively, the controller 13b can determine whether to end the process if it is detected that the user 200 is neither in contact with nor in proximity to the robot 100b, and not to end the process if it is detected that the user 200 is in contact with or in proximity to the robot 100b.

If it is determined in step S164 that the process is not to end (step S164, NO), the controller 13b performs the processing as of step S161 again. If it is determined in step S164 that the process is to end (step S164, YES), the controller 13b ends the process.

As described above, the controller 13b can perform the process for causing the robot 100b to guide breathing for the user 200.

### <Main Operation Effect of Robot 100b>

As described above, in the present embodiment, the expansion-contraction mechanism 18A includes the abdomen expansion-contraction mechanism 18a disposed in the abdomen 191 of the robot 100b and the back expansion-contraction mechanism 18b disposed in the back 192 of the robot 100b. The controller 13b expands and contracts at least one of the abdomen expansion-contraction mechanism 18a or the back expansion-contraction mechanism 18b based on the held state information M3.

For example, when the user 200 holds the robot 100b to be in contact with the back 192 of the robot 100b, the robot 100b cannot guide breathing for the user 200 if the abdomen 191 is expanded and contracted. The robot 100b expands and contracts at least one of the abdomen expansion-contraction mechanism 18a or the back expansion-contraction mechanism 18b in accordance with the held state information M3 obtained by detecting which of the abdomen 191 or the back 192 of the robot 100b is in contact with the user 200. With this, the robot 100b can surely guide breathing for the user 200 regardless of the state of the robot 100b when held by the user 200. Here, when the user 200 holds the robot 100b such that the side surface of the robot 100b is in contact with the user 200, the controller 13b may expand and contract both of the abdomen expansion-contraction mechanism 18a and the back expansion-contraction mechanism 18b. The effects other than the above are the same as those of the first embodiment.

Although the preferred embodiments have been described in detail above, the present invention is not limited to the above-described embodiments, and various modifications and substitutions can be made to the above-described embodiments without departing from the scope of the appended claims.

Additionally, the numerals, such as ordinal numbers and quantities used in the description of the above-described embodiments are all examples for specifically describing the technique of the present invention, and the present invention is not limited to the numerals described as examples. Additionally, the connection relationship between the constituent elements is an example for specifically describing the technique of the present invention, and the connection relationship for realizing the functions of the present invention is not limited thereto.

The robot according to the present embodiment is particularly suitable for use in promoting oxytocin secretion and providing comfort (sense of security or self-affirmation) to a working adult living alone, a senior person whose child has moved out, a frail elderly person who is a target of home healthcare, or the like. However, the present invention is not limited to this use, and can be used to provide comfort to various users.

Aspects of the present disclosure are as follows, for example.
<1> A robot configured to guide breathing for a user, the robot including:
   an exterior member;
   a first detector configured to acquire information on the breathing of the user; and
   a controller configured to control an operation of the robot so as to guide the breathing of the user to a predetermined state based on the information acquired by the first detector.
<2> The robot as described in <1>, wherein the first detector includes at least one of an electromagnetic wave sensor configured to acquire the information on the breathing of the user, using an electromagnetic wave, or an image sensor configured to acquire the information on the breathing of the user based on a captured image of the user.
<3> The robot as described in <1> or <2>, further including a second detector configured to output information on at least one of a pulse, a heartbeat, a blood pressure, or pulse pressure of the user,
   wherein the controller controls the operation of the robot so as to guide the breathing of the user to the predetermined state additionally based on the information acquired by the second detector.
<4> The robot as described in <3>, wherein the controller controls the operation of the robot so as to guide the breathing of the user to the predetermined state in accordance with information on a psychological state of the user, the information on the psychological state being obtained based on the information acquired by the second detector.
<5> The robot as described in any one of <1> to <4>, wherein the controller controls an operation of at least one of an expansion-contraction mechanism configured to expand and contract a trunk of the robot, a light emitter provided in a face of the robot, or a drive section including an arm connected to a robot main body of the robot so as to be relatively displaceable.
<6> The robot as described in [5], wherein the expansion-contraction mechanism includes an abdomen expansion-contraction mechanism disposed in an abdomen of the robot and a back expansion-contraction mechanism disposed in a back of the robot, and
   wherein the controller expands and contracts at least one of the abdomen expansion-contraction mechanism or the back expansion-contraction mechanism based on information on a state of the robot when held by the user.
<7> The robot as described in <5>, wherein the controller controls at least one of a blinking speed of the light emitter, a luminance of light from the light emitter, or a color of the light from the light emitter.
<8> The robot as described in <5>, wherein the controller controls at least one of an operation of wrapping the arm around a part of a body of the user, an operation of pressing a skin of the user by the arm, or an operation of stroking a part of the body of the user by the arm.
<9> The robot as described in any one of <1> to <8>, wherein the exterior member includes at least one of an elastic body or a porous body.

This application is based on and claims priority to Japanese Patent Application No. 2022-156760 filed in the Japan Patent Office on September 29, 2022, the entire contents of which are incorporated herein by reference.

### Description of reference symbols

1 trunk
2 head
2a right eye
2b left eye
2c mouth
2d right cheek
2e left cheek
3 arm
3a right arm
3b left arm
4 leg
4a right leg
4b left leg
5 nose
10 exterior member
11 camera (an example of a first detector and an example of an image sensor)
12 tactile sensor
13, 13a, 13b controller
14 vital sensor (an example of an electromagnetic wave sensor, an example of a first detector, and an example of a second detector)
141 microwave transmitter
142 microwave receiver
15 battery
16 trunk frame
17 trunk mounting base
18, 18A expansion-contraction mechanism
18a abdomen expansion-contraction mechanism
18b back expansion-contraction mechanism
21 first capacitive sensor
22 head frame
23 head mounting base
24 display
24a right-eye display
24b left-eye display
25 speaker
26 light (an example of a light emitter)
26a right-cheek light
26b left-cheek light
27 head connection mechanism
31 second capacitive sensor
32a right arm frame
32b left arm frame
33 right arm mounting base
34a right arm connection mechanism
34b left arm connection mechanism
35 servo motor
35a right arm servo motor
35b left arm servo motor
35c head servo motor
35d right leg servo motor
35e left leg servo motor
41a right leg wheel
41b left leg wheel
42a right leg frame
42b left leg frame
44a right leg connection mechanism
44b left leg connection mechanism
100, 100a, 100b robot
101 acquisition unit
102 communication control unit
103 storage unit
104 authentication unit
105 registration unit
106 start control unit
107, 107a motor control unit
108 detection unit
109 output unit
110 respiratory property information acquisition unit
111, 111a, 111b expansion-contraction control unit
112, 112a light emission control unit
113 psychological state information acquisition unit
114 held state information acquisition unit
130 correspondence relation information
131 CPU
132 ROM
133 RAM
134 HDD/SSD
135 device connection I/F
136 communication I/F
181 support
182 press drive section
183 rotation section
184 press section
191 abdomen
192 back
200 user
201 imaging light source
202 wavelength filter
203 lens
204 imaging element
A system bus
B biological information
C1 first capacitance signal
C2 second capacitance signal
F1a right shoulder frame
F2a right upper arm frame
F3a right elbow frame
F4a right forearm frame
F1b left shoulder frame
F2b left upper arm frame
F3b left elbow frame
F4b left forearm frame
F1c neck frame
F2c face frame
Im captured image
L irradiation light
Ms transmitted wave
Mr reflected wave
M1 respiratory property information
M2 psychological state information
M3 held state information
M1a right shoulder servo motor
M2a right upper arm servo motor
M3a right elbow servo motor
M4a right forearm servo motor
M1b left shoulder servo motor
M2b left upper arm servo motor
M3b left elbow servo motor
M4b left forearm servo motor
M1c neck servo motor
M2c face servo motor
N1, N1a, N2a, N2, N3 operation information
R reflected light
S tactile signal

## Claims

1. A robot configured to guide breathing for a user, the robot comprising:
an exterior member;
a first detector configured to acquire information on the breathing of the user; and
a controller configured to control an operation of the robot so as to guide the breathing of the user to a predetermined state based on the information acquired by the first detector.

2. The robot as claimed in claim 1, wherein the first detector includes at least one of an electromagnetic wave sensor configured to acquire the information on the breathing of the user, using an electromagnetic wave, or an image sensor configured to acquire the information on the breathing of the user based on a captured image of the user.

3. The robot as claimed in claim 1 or 2, further comprising a second detector configured to acquire information on at least one of a pulse, a heartbeat, a blood pressure, or pulse pressure of the user,
wherein the controller controls the operation of the robot so as to guide the breathing of the user to the predetermined state additionally based on the information acquired by the second detector.

4. The robot as claimed in claim 3, wherein the controller controls the operation of the robot so as to guide the breathing of the user to the predetermined state in accordance with information on a psychological state of the user, the information on the psychological state being obtained based on the information acquired by the second detector.

5. The robot as claimed in claim 1 or 2, wherein the controller controls an operation of at least one of an expansion-contraction mechanism configured to expand and contract a trunk of the robot, a light emitter provided in a head of the robot, or a drive section including an arm connected to a robot main body of the robot so as to be relatively displaceable.

6. The robot as claimed in claim 5,
wherein the expansion-contraction mechanism includes an abdomen expansion-contraction mechanism disposed in an abdomen of the robot and a back expansion-contraction mechanism disposed in a back of the robot, and
wherein the controller expands and contracts at least one of the abdomen expansion-contraction mechanism or the back expansion-contraction mechanism based on information on a state of the robot when held by the user.

7. The robot as claimed in claim 5,
wherein the expansion-contraction mechanism further includes a support, a press drive section, a rotation section, and a press section, and
wherein the rotation section has a rotation axis for causing the press section to oscillate back and forth so that the exterior member of the trunk expands and contracts.

8. The robot as claimed in claim 5, wherein the controller controls at least one of a blinking speed of the light emitter, a luminance of light from the light emitter, or a color of the light from the light emitter.

9. The robot as claimed in claim 5, wherein the controller controls at least one of an operation of wrapping the arm around a part of a body of the user, an operation of pressing a skin of the user by the arm, or an operation of stroking a part of the body of the user by the arm.

10. The robot as claimed in claim 1 or 2, wherein the exterior member includes at least one of an elastic body or a porous body.
